(19) Europäisches Patentamt
European Patent Office
Office européen des brevets

(11) Numéro de publication: **0 020 281**
**B1**

(12) **FASCICULE DE BREVET EUROPEEN**

(45) Date de publication du fascicule du brevet: **01.04.87**

(51) Int. Cl.$^4$: **C 07 D 263/58**

(21) Numéro de dépôt: **80420053.3**

(22) Date de dépôt: **07.05.80**

(54) **Préparation de benzoxazolone.**

(30) Priorité: **08.05.79 FR 7912140**

(43) Date de publication de la demande:
**10.12.80 Bulletin 80/25**

(45) Mention de la délivrance du brevet:
**01.04.87 Bulletin 87/14**

(84) Etats contractants désignés:
**AT BE CH DE FR GB IT LI LU NL SE**

(56) Documents cités:
**AU-A- 205 525**
**FR-A-1 269 067**

**Bull. Soc. Chim. (1954), pages 393 - 5**

(73) Titulaire: **RHONE-POULENC AGROCHIMIE**
**14-20, rue Pierre Baizet**
**F-69009 Lyon (FR)**

(72) Inventeur: **Querou, Yvon**
**2, Rue Zilina**
**F-92000 - Nanterre (FR)**

(74) Mandataire: **Brachotte, Charles et al**
**RHONE-POULENC AGROCHIMIE BP 9163-09**
**F-69263 Lyon Cedex 1 (FR)**

**Le dossier contient des informations techniques présentées postérieurement au dépôt de la demande et ne figurant pas dans le présent fascicule.**

EP 0 020 281 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne un procédé de préparation de composé hétérocyclique à partir de phénol. Plus spécifiquement l'invention concerne un procédé de préparation de benzoxazolone à de chlorophénol.

La benzoxazolone a pour formule:

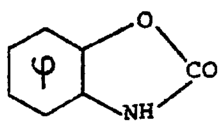

Il s'agit d'un produit intermédiaire utile pour la synthèse d'autres produits, par exemple l'insecticide connu sous le nom de phosalone. Cette benzoxazolone est quelquefois appelée, notamment en langue anglaise, benzoxazolinone.

Le brevet FR—A—1 269 067 et Lespagnol dans Bull. Soc. Chim. (1954) p. 393—5 décrivent la préparation de benzoxazolone par chauffage d' O. aminophénol et d'usée. Le brevet AU—205525 décrit las préparation d' O. aminophénol par réaction d'ammoniac sur 1'O. chlorophénol.

Un but de l'invention est de fournir un procédé simple de préparation de la benzoxazolone à partir de réactifs simples, et plus particulièrement à partir d'orthochlorophénol. Il a donc été trouvé un procédé de préparation de benzoxazolone à partir d'orthochlorophénol caractérisé en ce que (dans une première étape) de l'orthochlorophénol est chauffé sous pression d'ammoniac et puis que (dans une deuxième étape) de lurée est ajoutée au mélange réactionnel obtenu au cours de la première étape et que l'ensemble est chauffé sous pression atmosphérique en présence d'eau. Le procédé est réalisé en présence d'un catalyseur à base de cuivre, tels que les sels cuivreux ou cuivriques, notamment les halogénures, sulfates, phosphates, acétates, propionates, acétylacétonates (le chlorure cuivreux est préféré), ainsi que les oxydes, (notamment l'oxyde cuivreux) et le cuivre métallique.

La quantité de catalyseur comprise dans le milieu réactionnel est généralement comprise entre 0,5 et 20% en poids par rapport à l'orthochlorophénol, de préférence comprise entre 2 et 10%. Des quantités sortant de ces limites peuvent, cependant être utilisées, sans toutefois que cela présente un avantage économique important.

La première étape réactionnelle est donc effectuée sous pression d'ammoniac. Autrement dit, il s'agit d'un chauffage d'un milieu réactionnel liquide, surmonté par une atmosphère sous pression contenant de l'ammoniac. Il s'agit de préféférence de $NH_3$ ajouté, sous pression, dans l'atmosphère surmontant le milieau réactionnel. La pression totale est généralement comprise entre 1 et 60 bars (pressions relatives), de préférence entre 3 et 40 bars. Des pressions supérieures, allant par exemple jusqu'à 150 bars, peuvent être aussi utilisées.

Dans cette atmosphère contenant de l'ammoniac et surmontant le milieu réactionnel lors de la première étape, l'ammoniac ($NH_3$) est de préférence essentiellement à l'état pur mais peut aussi se trouver à une pression partielle supérieure à 50% de la pression totale, ou mieux supérieure à 90% de cette pression. Cet ammoniac provient le plus souvent, soit d'une alimentation extérieure, soit d'une introduction préalable dans le réacteur, soit d'une conjonction de ces facteurs. La température du milieu réactionnel est généralement comprise, dans la première étape, entre 100 et 250°C, de préférence entre 140 et 230°C.

Cette première étape réactionnelle peut être effectuée en présence de solvants minéraux ou organiques, mais l'on préfère généralement opérer en masse. Cette masse est normalement liquide, surtout à la température réactionnelle.

La durée de cette première étape réactionnelle est évidemment variable selon les conditions opératoires. L'homme de l'art pourra par de simples essais de routine déterminer la durée optimale; généralement on poursuit cette étape réactionnelle jusqu'à ce que le teneur en orthochlorophénol ne varie plus substantiellement ou, autrement dit, jusqu'à ce que le taux de transformation de l'orthochlorophénol ait sensiblement atteint son maximum, la simple dégradation thermique et/ou chimique étant mise à part.

Comme cela a été indiqué le procédé de l'invention comprend 2 étapes réactionnelles; ces 2 étapes sont effectivement distinctes notamment par le fait que la première est affectuée sous pression alors le seconde est simplement effecturée sous pression atmosphérique. Cependant, hormis cette distinction, ces 2 étapes sont assez voisines sur le plan pratique et elles peuvent être effectuées l'une à la suite de l'autre, simplement par un petit changement de conditions opératoires et avec adjonction d'urée avant la 2ème étape, mais sans qu'il soit vraiment nécessaire, entre les 2 étapes réactionnelles, de changer le milieu réactionnel de réacteur ou de la soumettre à des traitements particuliers. Cela fait ressortir que malgré la présence apparente de 2 étapes reactionnelles, le procédé de l'invention est d'une grande simplicité et d'une grande commodité de mise en oeuvre. Cette simplicité et cette commodité sont pratiquement aussi grandes que s'il s'agissait d'un procédé ne comportant strictement qu'une étape réactionnelle.

La second étape réactionnelle s'effectue sous pression atmosphérique, de préférence sous atmosphère libre et en présence d'urée. Etant donné la volatilité d l'ammoniac, le chauffage sous pression atmosphérique est équivalent à un chauffage en absence totale ou presque totale d'ammoniac.

Un peu d'ammoniac peut cependant être présent, notamment sous l'effet d'une certaine décomposition de l'urée mais même dans ce cas, l'ammoniac s'échappe du milieu à chaud.

Le rapport molaire de l'urée mise en oeuvre au cours de la deuxième étape de la réaction par rapport à l'orthochlorophénol (initialement) mis

en oeuvre est habituellement compris entre 1 et 15, de préférence entre 1,2 et 8.

Le température réactionnelle au cours de cette seconde étape est généralement comprise entre 80 et 220°C de préférence entre 110 et 190°C.

Le seconde étape réactionnelle et effectuée en présence d'eau. Toutefois étant donné la température et la pression, l'eau a tendance à s'évaporer plus ou moins rapidement en sorte que, selon une variante, on alimente le milieu réactionnel en eau liquide de manière continue et, éventuellement, on récupère et condense la vapeur d'eau issue du milieu réactionnel. Selon de telles modalités, il est avantageux d'alimenter en eau le milieu réactionnel avec un débit horaire inférieur à 20% en poids de ce milieu réactionnel. En fin de réaction, on isole la benzoxazolone par tout moyen connu en soi. Selon une modalité opérationnelle très avantageuse, on précipite la benzoxazolone par de l'eau éventuellement acidifiée; la benzoxazolone peut être purifiée par tout moyen connu en soi, par exemple par recristallisation, ou lavage avec des solvants organiques.

Le procédé de l'invention est spécialement avantageux en raison des bons résultats obtenus, tant en ce qui concerne le taux de transformation de l'orthochlorophénol que les rendements en benzoxazolone et que la simplicité et la commodité de mise en oeuvre.

Les exemples suivants, donnés à titre non limitatif, illustrent l'invention et montrent comment elle peut être mise en oeuvre.

Dans ces exemples T T désigne le taux de transformation de l'orthochlorophénol et R T désigne le rendement en benzoxazolone par rapport à l'orthochlorophénol transformé.

Exemple 1

Dans un autoclave de 125 $cm^3$ en acier inoxydable muni d'un système d'agitation, on charge:

— 12,85 g d'orthochlorophénol

— 0,715 g de $Cu_2O$

On purge l'autoclave à l'ammoniac puis on charge 14,7 g d'ammoniac ($NH_3$). On chauffe à 140°C pendant 8 heures. La pression se stabilise à 40 bars en début de chauffage et à 20 bars en fin de chauffage. On refroidit à 125°C, laisse l'ammoniac s'échapper. On ajoute 6,12 g d'urée et 1 g d'eau puis chauffe à pression atmosphérique à 140°C pendant 2 heures puis à 150°C pendant 2 heures (à la même pression). On refroidit à 120°C, ajoute 100 $cm^3$ d'HCl en solution aqueuse N.

La benzoxazolone précipitée est filtrée et, est ainsi obtenue avec un R T de 53% et un T T de 65%.

Exemple 2

Dans l'appareillage utilisé à l'exemple 1, on charge:

— 12,85 g d'orthochlorophenol

— 0,99 g de $Cu_2Cl_2$

On purge l'autoclave à l'ammoniac puis charge 27 g d'ammoniac.

On chauffe à 170°C pendant 3 heures la pression étant d'environ 120 bars. On refroidit à 125°C et laisse l'ammoniac s'échapper; on charge 5,4 g d'urée et 1 g d'eau. Puis, en opérant comme à l'exemple 1, on obtient la benzoxazolone avec un R T de 67% et un T T de 69%.

Exemple 3

On reproduit l'exemple 2, mais en chauffant à 170°C pendant 8 heures (au lieu de 3 heures) et en chargeant 7,7 g d'urée (au lieu de 5,4 g), on obtient ainsi la benzoxazolone avec un R T de 70% et un T T de 93%.

Exemple 4

Dans un autoclave de 140 $cm^3$ revêtu intérieurement de polytétrafluoroéthylène, on charge:

— 7,2 g d'orthochlorophénol

— 0,28 g de chlorure cuivreux.

On ferme l'autoclave, purge à l'aide de $NH_3$ et on charge 7 g de $NH_3$. On chauffe à 180°C pendant 6 heures. La pression se stabilise initialement à environ 40 bars (pression relative) puis descend progressivement jusqu'à environ 26 bars en fin de réaction.

On refroidit à 120°C, ouvre l'autoclave, puis ajoute 16,8 g d'urée et 0,6 $cm^3$ d'eau. On chauffe sans distiller sous pression atmosphérique pendant 2 h à 150°C puis 2h à 180°C. On refroidit à 120°C, ajoute 60 $cm^3$ de solution aqueuse d'acide chlorhydrique N, refroidit à 20°C et extrait à l'acétate d'éthyle. Dans la phase organique on obtient la benzoxazolone avec un RT de 56% et un T T de 99%.

**Revendications**

1. Procédé de préparation de benzoxazolone caractérisé en ce que de l'orthochlorophénol est chauffé sous pression d'ammoniac puis de l'urée est ajoutée au mélange réactionnel et que l'ensemble est chauffé sous pression atmosphérique et en présence d'eau, le procédé étant effectué en présence d'un catalyseur à base de cuivre.

2. Procédé selon la revendication 1 caractérisé en ce que le catalyseur à base de cuivre est un catalyseur à base de sel cuivreux.

3. Procédé selon la revendication 2 caractérisé en ce que le catalyseur est à base de chlorure cuivreux.

4. Procédé selon l'une des revendications 1 à 3 caractérisé en ce que la pression, dans la première étape réactionnelle, est comprise entre 1 et 60 bars.

5. Procédé selon la revendication 4 caractérisé en ce que la pression, dans la première étape réactionnelle, est comprise entre 3 et 40 bars.

6. Procédé selon l'une des revendications 4 ou 5 caractérisé en ce que la pression partielle d'ammoniac de l'atmosphère surmontant le milieu réactionnel lors de la première étape réactionnelle est supérieure à 50% de la pression totale.

7. Procédé selon la revendication 6 caractérisé en ce que la pression partielle d'ammoniac, au cours de la première étape réactionnelle, est supérieure à 90% de la pression totale.

8. Procédé selon l'une des revendications 1 à 7

caractérisé en ce que le rapport molaire de l'urée mise en oeuvre, par rapport à l'orthochlorophénol mis en oeuvre, est compris entre 1 et 15.

9. Procédé selon la revendication 8 caractérisé en ce que le rapport urée/orthochlorophénol est compris entre 1,2 et 8.

10. Procédé selon l'une des revendications 1 à 9 caractérisé en ce que la température du milieu réactionnel lors de la première étape réactionnelle est comprise entre 100 et 250°C.

11. Procédé selon la revendication 10 caractérisé en ce que la température du milieu réactionnel lors de la première étape réactionnelle est comprise entre 140 et 230°C.

12. Procédé selon l'une des revendications 1 à 11 caractérisé en ce que la température de milieu réactionnel lors de la deuxième étape réactionnelle est comprise entre 80 et 220°C.

13. Procédé selon la revendication 12 caractérisé en ce que la température du milieu réactionnel lors de la deuxième étape réactionnelle est comprise entre 110 et 190°C.

14. Procédé selon l'une des revendications 1 à 13 caractérisé en ce que le teneur en catalyseur du milieu réactionnel est comprise entre 0,5 et 20% en poids par rapport à l'orthochlorophénol.

15. Procédé selon l'une des revendications 1 à 14 caractérisé en ce que la teneur en catalyseur est comprise entre 2 et 10% en poids par rapport à l'orthochlorophénol.

**Patentansprüche**

1. Verfahren zur Herstellung von Benzoxazolon, dadurch gekennzeichnet, daß Orthochlorphenol unter Ammoniakdruck erhitzt wird, dann Harnstoff zu dem Reaktionsgemisch zugegeben wird und das Ganze unter Atmosphärendruck und in Gegenwart von Wasser erhitzt wird, wobei das Verfahren in Gegenwart eines Katalysators auf Kupferbasis durchgeführt wird.

2. Verfahren gemäß Anspruch 1, dadurch gekennzeichnet, daß der Katalysator auf Kupferbasis ein Katalysator auf Basis eines Cuprosalzes ist.

3. Verfahren gemäß Anspruch 2, dadurch gekennzeichnet, daß der Katalysator auf Basis von Cuprochlorid ist.

4. Verfahren gemäß einem der Ansprüche 1 bis 3, dadurch gekennzeichnet, daß der Druck in der ersten Reaktionsstufe zwischen 1 und 60 bar beträgt.

5. Verfahren gemäß Anspruch 4, dadurch gekennzeichnet, daß der Druck in der ersten Reaktionsstufe zwischen 3 und 40 bar beträgt.

6. Verfahren gemäß einem der Ansprüche 4 oder 5, dadurch gekennzeichnet, daß der Ammoniakpartialdruck der das Reaktionsmilieu überlagernden Atmosphäre während der ersten Reaktionsstufe oberhalb 50% des Gesamtdrucks liegt.

7. Verfahren gemäß Anspruch 6, dadurch gekennzeichnet, daß der Ammoniakpartialdruck während der ersten Reaktionsstufe oberhalb 90% des Gesamtdrucks liegt.

8. Verfahren gemäß einem der Ansprüche 1 bis 7, dadurch gekennzeichnet, daß das Molverhältnis des eingesetzten Harnstoffs in bezug auf eingesetztes Orthochlorphenol zwischen 1 und 15 beträgt.

9. Verfahren gemäß Anspruch 8, dadurch gekennzeichnet, daß das Verhältnis Harnstoff/Orthochlorphenol zwischen 1,2 und 8 beträgt.

10. Verfahren gemäß einem der Ansprüche 1 bis 9, dadurch gekennzeichnet, daß die Temperatur des Reaktionsmilieus während der ersten Reaktionsstufe zwischen 100 und 250°C beträgt.

11. Verfahren gemäß Anspruch 10, dadurch gekennzeichnet, daß die Temperatur des Reaktionsmilieus während der ersten Reaktionsstufe zwischen 140 und 230°C beträgt.

12. Verfahren gemäß einem der Ansprüche 1 bis 11, dadurch gekennzeichnet, daß die Temperatur des Reaktionsmileus während der zweiten Reaktionsstufe zwischen 80 und 220°C beträgt.

13. Verfahren gemäß Anspruche 12, dadurch gekennzeichnet, daß die Temperatur des Reaktionsmilieus während der zweiten Reaktionsstufe zwischen 110 und 190°C beträgt.

14. Verfahren gemäß einem der Ansprüche 1 bis 13, dadurch gekennzeichnet, daß der Gehalt an Katalysator des Reaktionsmilieus zwischen 0,5 und 20 Gew.% in bezug auf das Orthochlorphenol beträgt.

15. Verfahren gemäß einem der Ansprüche 1 bis 14, dadurch gekennzeichnet, daß der Gehalt an Katalysator zwischen 2 und 10 Gew.% in bezug auf das Orthochlorphenol beträgt.

**Claims**

1. A process for the preparation of benzoxazolone, characterized in that (1) *ortho*-chlorophenol is heated under ammonia pressure and (2) urea is added to the reaction mixture and the resulting mixture is heated under atmospheric pressure and in the presence of water, said process being carried out in the presence of a based on copper catalyst.

2. A process according to claim 1 characterized in that the catalyst is a cuprous salt.

3. A process according to claim 1 characterized in that the catalyst is cuprous chloride.

4. A process according to any one of claims 1 to 3 characterized in that the pressure in the first reaction step is from 1 to 60 bars.

5. A process according to claim 4 characterized in that pressure in the first reaction step is from 3 to 40 bars.

6. A process according to claim 4 or 5 characterized in that the partial pressure of ammonia in the atmosphere surmounting the reaction medium in the first reaction step is more than 50% of the total pressure.

7. A process according to claim 6 characterized in that the partial pressure of ammonia in the first reaction step is more than 90% of the total pressure.

8. A process according to any one of claims 1 to 7 characterized in that the molar ratio of the urea

used to the *ortho*-chlorophenol used is from 1 to 15.

9. A process according to claim 8 characterized in that the ratio urea/*ortho*-chlorophenol is from 1.2 to 8.

10. A process according to any one of claims 1 to 9 characterized in that the temperature of the reaction medium in the first reaction step is from 100 to 250°C.

11. A process according to claim 10 characterized in that the temperature of the reaction medium in the first reaction step is from 140 to 230°C.

12. A process according to any one of claims 1 to 11 characterized in that the temperature of the reaction medium in the second reaction step is from 80 to 220°C.

13. A process according to claim 12 characterized in that the temperature of the reaction medium in the second reaction step is from 110 to 190°C.

14. A process according to any one of claims 1 to 13 characterized in that the proportion of catalyst in the reaction medium is from 0.5 to 20% by weight, relative to the *ortho*-chlorophenol.

15. A process according to any one of claims 1 to 14 characterized in that the proportion of catalyst is from 2 to 10% by weight, relative to the *ortho*-chlorophenol.